(19)

**Europäisches Patentamt**

**European Patent Office**

**Office européen des brevets**

(11) **EP 4 014 836 A1**

(12) **EUROPEAN PATENT APPLICATION**

(43) Date of publication:
**22.06.2022 Bulletin 2022/25**

(21) Application number: **20214003.4**

(22) Date of filing: **15.12.2020**

(51) International Patent Classification (IPC):
$A61B\ 5/00^{(2006.01)}$   $A61N\ 1/04^{(2006.01)}$
$A61N\ 1/36^{(2006.01)}$   $A61N\ 7/00^{(2006.01)}$
$A61N\ 5/00^{(2006.01)}$   $A61N\ 2/00^{(2006.01)}$
$A61B\ 5/055^{(2006.01)}$   $A61B\ 5/242^{(2021.01)}$
$A61B\ 5/377^{(2021.01)}$   $A61B\ 6/00^{(2006.01)}$
$A61B\ 8/06^{(2006.01)}$   $G16H\ 30/40^{(2018.01)}$

(52) Cooperative Patent Classification (CPC):
**A61N 1/0456; A61B 5/0042; A61B 5/055;
A61B 5/4064; A61B 5/489; A61B 6/037;
A61B 6/501; A61B 6/506; A61B 6/507;
A61B 6/5217; A61B 8/06; A61B 8/0808;
A61B 8/0816; A61B 8/085; A61B 8/5223;**   (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
- **HUIJBERS, Willem
  5656 AE Eindhoven (NL)**
- **VAN DOOREN, Marieke
  5656 AE Eindhoven (NL)**

- **VAN EE, Raymond
  5656 AE Eindhoven (NL)**
- **LAMERICHS, Rudolf Mathias Johannes Nicolaas
  5656 AE Eindhoven (NL)**
- **LEUFKENS, Timmy Robertus Maria
  5656 AE Eindhoven (NL)**
- **MATTERS, Marco
  5656 AE Eindhoven (NL)**
- **WESTERINK, Joanne Henriëtte Desirée Monique
  5656 AE Eindhoven (NL)**
- **DENISSEN, Adrianus Johannes Maria
  5656 AE Eindhoven (NL)**

(74) Representative: **Philips Intellectual Property &
Standards
High Tech Campus 52
5656 AG Eindhoven (NL)**

(54) **METOHD AND SYSTEM FOR CALIBRATION OF BRAIN HEMODYNAMICS**

(57)   In order to enable more accurate calibration of brain, there is provided a system for creating a hemodynamic brain atlas for calibration of brain hemodynamics. The system comprises a non-invasive transcranial neurostimulation device, a non-invasive neuromonitoring device, and a computing device. The non-invasive transcranial neurostimulation device is configured to induce neuronal activity to evoke a hemodynamic response in a target region of interest (ROI) of a brain of a human subject. The non-invasive neuromonitoring device is configured to monitor the evoked hemodynamic response in the target ROI. The computing device configured to determine a set of parameters representing a hemodynamic response function, HRF, of the evoked hemodynamic response in the target ROI, and to associate the set of parameters of the HRF with the target ROI to form the hemodynamic brain atlas.

Fig. 2A

**(Cont. next page)**

EP 4 014 836 A1

(52) Cooperative Patent Classification (CPC): (Cont.)
**A61N 1/36014; A61N 1/36025; A61N 1/36034;
A61N 2/006; G16H 20/30; G16H 30/40;
G16H 50/20;** A61B 6/5235; A61B 6/54;
A61B 8/5207; A61N 2007/0026; A61N 2007/0078;
A61N 2007/0095; G16H 20/10

**Description**

FIELD OF THE INVENTION

The present invention relates to calibration of brain hemodynamics, and in particular to a system for creating a hemodynamic brain atlas for calibration of brain hemodynamics, a device for calibration of brain hemodynamics, a neuromonitoring system, a method for creating a hemodynamic brain atlas, a computer-implemented method for calibration of brain hemodynamics, and a computer program element.

BACKGROUND OF THE INVENTION

Functional magnetic resonance imaging (fMRI) is limited by the use of a canonical hemodynamic response function (HRF) across the brain. The use of one single canonical HRF, which is the same across the entire brain and across individuals, may be a limitation. The limitation of canonical HRF can be mitigated partially, by estimation of HRF parameters in response to events (HRF calibration). However, this calibration can only be done in brain regions that have a tight coupling between events and evoked neuronal activity, i.e. the visual cortex.

SUMMARY OF THE INVENTION

There may be a need to improve accuracy of brain hemodynamics calibration.

The object of the present invention is solved by the subject-matter of the independent claims, wherein further embodiments are incorporated in the dependent claims. It should be noted that the following described aspects of the invention apply also for the system for creating a hemodynamic brain atlas for calibration of brain hemodynamics, the device for calibration of brain hemodynamics, the neuromonitoring system, the method for creating a hemodynamic brain atlas, the computer-implemented method for calibration of brain hemodynamics, and the computer program element.

According to a first aspect of the present invention, there is provided a system for creating a hemodynamic brain atlas for calibration of brain hemodynamics. The system comprises a non-invasive transcranial neurostimulation device, a non-invasive neuromonitoring device, and a computing device. The non-invasive transcranial neurostimulation device is configured to induce neuronal activity to evoke a hemodynamic response in a target region of interest (ROI) of a brain of a human subject. The non-invasive neuromonitoring device is configured to monitor the evoked hemodynamic response in the target ROI. The computing device configured to determine a set of parameters representing a hemodynamic response function, HRF, of the evoked hemodynamic response in the target ROI, and to associate the set of parameters of the HRF with the target ROI to form the hemodynamic brain atlas.

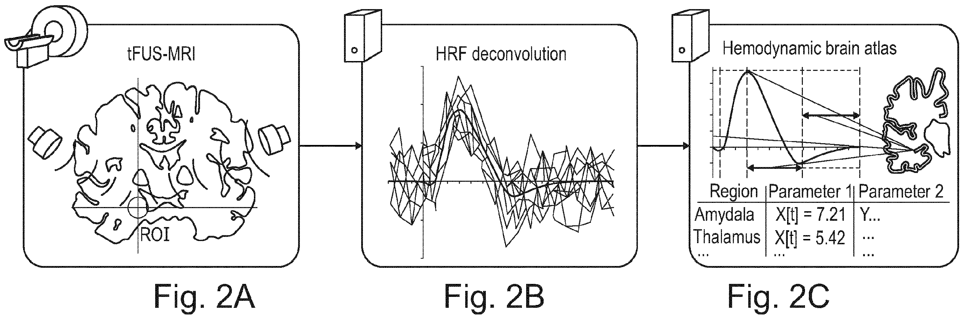

In other words, it is proposed to use transcranial neurostimulation, such as focused ultrasound stimulation (tFUS), transcranial electrical stimulation (tDCS/tACS), and transcranial magnetic stimulation (TMS), to evoke neural activity and to subsequently estimate the HRF across various other brain regions. Neurostimulation has the advantage to be able to target any specified brain region, but also to be able to target multiple different brain regions. This may enable more accurate calibration of fMRI, not only in individuals, but also for specific brain regions. It is also proposed to develop a hemodynamic brain atlas that comprises one or more brain regions. Each brain region is associated with a respective hemodynamic response function (HRF) represented by a set of parameters. Thus, the hemodynamic brain atlas comprises a plurality of regionally calibrated HRFs. An exemplary hemodynamic brain atlas is illustrated in Fig. 2C. This atlas may improve the accuracy of fMRI measurement and could be extended by other methods of neurostimulation and neuromonitoring.

The target ROI may be anatomically, functionally, or clinically defined. The target ROI may be anatomically defined as the motor cortex, visual cortex, amygdala, a volume at an atlas coordinate, or a specific part of a brain region in the cortex (e.g. cortical layer IV of MT in the left hemisphere), sub-cortical region (e.g. substantia nigra, thalamus, hippocampus), or a region in the cerebellum. The target ROI can be functionally defined using and localizer task in the fMRI (motor, language, etc.), using functional connectivity, a functional atlas or using a PET (positron emission tomography) tracer. The target ROI may be clinically, for example as the foci of epileptic seizures or a stroke.

The set of parameters representing HRF may include one or more of peak amplitude, trough amplitude, time to peak, time to fall, rise slope, fall slope, FWHM (full width at half maximum, or area under the curve), etc.

According to an embodiment of the present invention, the non-invasive transcranial neurostimulation device is configured to evoke a plurality of hemodynamic responses in a plurality of target ROIs, each hemodynamic response being evoked in a respective target ROI. The computing device is configured to determine, for each evoked hemodynamic response, a respective set of parameters representing the corresponding HRF and to associate each set of parameters with the corresponding target ROI to form the hemodynamic brain atlas.

For example, the non-invasive transcranial neurostimulation device may target multiple different brain regions consecutively or at the same time by multiple transducers.

According to an embodiment of present invention, the non-invasive neuromonitoring device is configured to monitor a further evoked hemodynamic response in one or more brain regions that have sufficiently strong excitatory connectivity with the target ROI. The computing device is configured to determine a set of parameters representing an HRF of the further evoked hemodynamic response in the one or more brain regions, and to associate the set of parameters with the one or more brain regions to form the hemodynamic brain atlas.

Accordingly, the HRF is not only estimated in the stimulated ROI, but also in other regions that might show a consistent BOLD response after stimulation.

For example, tFUS might also evoke a neuronal, and ensure BOLD response, in a distal brain region with strong excitatory connectivity.

According to an embodiment of the present invention, the system further comprises a sensory stimulation device configured to apply at least one sensory stimulus over a human subject to induce neuronal activity to evoke a hemodynamic response in the target ROI. The computing device is configured to perform a comparison between the hemodynamic response evoked by the non-invasive transcranial neurostimulation device and the hemodynamic response evoked by the sensory stimulation device to correct a potential neurostimulation-induced confound on the HRF.

In other words, this system may also correct for potential neurostimulation-induced confounds on the HRF, for example via a comparison between tFUS-evoked HRFs and sensory/motor evoked HRF's. For one single distinct brain region, the deconvolution of the neurostimulation-evoked HRF may be compared to deconvolution of a sensory-evoked HRF.

The sensory stimulus may include one or more of visual stimulus, auditory stimulus, tactile stimulus, heat stimulus, etc.

According to an embodiment of the present invention, the non-invasive transcranial neurostimulation device is configured to evoke a sequence of hemodynamic responses in the target ROI. The non-invasive neuromonitoring device is configured to monitor the sequence of evoked hemodynamic responses in the target ROI. The computing device is configured to perform a comparison among the sequence of evoked hemodynamic responses to correct a potential neurostimulation-induced confound on the HRF.

In an example, the non-invasive neuromonitoring device may be an MRI. The MRI may use specific fMRI sequences to better separate the influence of changes in cerebral blood flow and BOLD, such as vascular space occupancy (VASO) depended BOLD fMRI, or a FMR sequence that allows better estimation of the influence of temperature changes and BOLD.

According to an embodiment of the present invention, the target ROI is selected from a set of calibration brain regions. An HRF in other brain regions of the brain is derivable from an HRF in the set of calibration brain regions.

An optimal set of calibration regions may be defined by exploring the regularities across brain regions. Although the HRF differs across brain regions, there are regularities (for example between hemispheres). Once a sufficient number of patients have been mapped into a hemodynamic brain atlas, these regularities can be exploited, and an optimal set of calibration regions can be defined.

Using a small set of individually calibration regions, for example one in each lobe, the method may infer a whole-brain hemodynamics.

According to an embodiment of the present invention, the non-invasive transcranial neurostimulation device comprises one or more of: transcranial functional ultrasound stimulation (tFUS), transcranial electrical stimulation (tDCS/tACS), and transcranial magnetic stimulation (TMS).

According to an embodiment of the present invention, the non-invasive neuromonitoring device comprises one or more of: a device for neuromonitoring of brain hemodynamics, and a device for measuring electrical signals produced by neurons to measure brain activity.

The hemodynamic neuroimaging device may include e.g. MRI, low intensity focused ultrasound imaging (LIFU), and near-infrared spectroscopy (NIRS).

Other methods of non-invasive neuromonitoring, e.g. electroencephalography (EEG), magnetoencephalography (MEG), Optically-Pumped Magnetometers (OPM), or motor-evoked responses, may be used to estimate the amount of neuronal activity induced by the neurostimulation.

According to a second aspect of the present invention, a device for calibration of brain hemodynamics. The device comprises an input unit, a processing unit, and an output unit. The input unit is configured to receive (i) a hemodynamic response in a brain region of interest (ROI) of a human subject acquired by a non-invasive neuromonitoring device and (ii) a hemodynamic brain atlas. The hemodynamic brain atlas comprises one or more brain regions, each brain region being associated with a respective hemodynamic response function, HRF, represented by a set of parameters. The processing unit is to calibrate the acquired hemodynamic response with the hemodynamic brain atlas. The output unit is configured to output the calibrated hemodynamic response.

In other words, a system is proposed for deploying the hemodynamic brain atlas, using the newly and correctly calibrated HRF, e.g. on a MRI system.

This hemodynamic brain atlas may include an average from a group of healthy adults, from a specified patient group, and/or from a single patient for whom many brain regions have been calibrated. This external atlas might also include HRF parameters that have been derived from different individuals for various brain regions.

According to an embodiment of the present invention, the hemodynamic brain atlas is derivable from one or more of: previous HRF measurements of the human subject, previous HRF measurements of a group of healthy adults, optionally stratified by age, sex or other factors, previous HRF measurements of a specified patient group, and previous HRF measurements of a single patient for whom may brain regions have been calibrated.

According to an embodiment of the present invention, the target ROI is different from the one or more brain regions of the hemodynamic brain atlas.

The calibrated HRF may be applied to other brain regions than those that showed a response during calibration. For example, a hemodynamic brain atlas with a set of calibrated ROIs from the left hemisphere may be used to estimate brain activity in the contra-lateral hemisphere.

According to a third aspect of the present invention, there is provided a neuromonitoring system. The neuromonitoring system comprises a non-invasive neuromonitoring device for acquiring a hemodynamic response in a region of interest (ROI) of a brain of a human subject, and a device according to the second aspect and any associated example for calibrating the acquired hemodynamic response.

According to a fourth aspect of the present invention, there is provided a method for creating a hemodynamic brain atlas for calibration of brain hemodynamics, comprising:

- inducing, by a non-invasive transcranial neurostimulation device, neuronal activity to evoke a hemodynamic response in a target region of interest, ROI, of a brain of a human subject;
- monitoring, by a non-invasive neuromonitoring device, the evoked hemodynamic response in the target ROI;
- determining, by a computing device, a set of parameters representing a hemodynamic response function, HRF, of the evoked hemodynamic response in the target ROI; and
- associating, by the computing device, the set of parameters of the HRF with the target ROI to form the hemodynamic brain atlas.

According to a fifth aspect of the present invention, there is provided a computer-implemented method for calibration of brain hemodynamics, comprising:

- receiving, by an input unit, (i) a hemodynamic response in a region of interest, ROI, of a brain of a human subject acquired by a non-invasive neuromonitoring device and (ii) a hemodynamic brain atlas,
  wherein the hemodynamic brain atlas comprises one or more brain regions, each brain region being associated with a respective hemodynamic response function, HRF, represented by a set of parameters;
- calibrating, by a processing unit, the acquired hemodynamic response with the hemodynamic brain atlas; and
- outputting, by an output unit, the calibrated hemodynamic response.

According to another aspect of the present invention, there is provided a computer program element for controlling a system according to the first aspect and any associated example or a device according to the second aspect and any associated example, which when being executed by a processor is configured to carry out the method according to the third or fourth aspect.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.

Fig. 1 schematically shows an example of a system for creating a hemodynamic brain atlas for calibration of brain hemodynamics

Figs. 2A-2C schematically shows an operation example of using the tFUS-MRI system for creating a hemodynamic brain atlas.

Fig. 3 schematically shows a further example of a system for creating a hemodynamic brain atlas for calibration of brain hemodynamics.

Fig. 4 schematically illustrates a device for calibration of brain hemodynamics.

Fig. 5 schematically shows an example of a neuromonitoring system.

Fig. 6 shows a flowchart of a method for creating a hemodynamic brain atlas for calibration of brain hemodynamics.

Fig. 7 shows a flowchart of a method for calibration of brain hemodynamics.

DETAILED DESCRIPTION OF EMBODIMENTS

Functional Magnetic Resonance Imaging (fMRI) is a non-invasive tool used to measure neuronal activity in response to a stimulus, task or under resting-state conditions. Unfortunately, fMRI does not directly measure the activity of neurons, i.e. action potentials of single cell neurons, but relies on the coupling between neuronal metabolism and blood flow. Functional MRI sequences typically employ the blood oxygen level-dependent (BOLD) contrast to estimate brain activity. Hence, fMRI depends on the response of blood vessels in the brain, and is therefore limited, and temporally filtered, by the hemodynamics of the brain's vasculature.

Event-related fMRI studies have identified a canonical hemodynamic response function (HRF) of the brain. An event, e.g. the presentation of short visual stimulus will evoke almost instantaneous neuronal activity in the visual cortex. In contrast to neuronal activity, the HRF is delayed. In the visual cortex, the stimulus evokes a delayed increase in BOLD signal, due to the dilation of the blood vessels and a large influx of oxygenated blood. This large increase in signal is the main source of fMRI. The HRF typically peaks at approximately 6 second after visual stimulation. After about 15-20 seconds, the HRF slowly returns to baseline. The HRF may be used to estimate brain activity in task fMRI, by convolution with the stimuli or task events. Furthermore, the HRF may be used to deconvolve fMRI time series and estimate neuronal events and task-evoked changes in brain connectivity. In sum, the HRF is acritical component of fMRI, in order to link changes in BOLD signal to events and vice versa.

The use of one single canonical HRF is considered a limitation, as it is the same across the entire brain and across individuals. Further, it has been shown that the HRF function is also age-dependent.

The above-mentioned limitation, e.g. the use of one single canonical HRF across the entire brain and across individuals, may be mitigated partially by estimation of HRF parameters in response to certain events, i.e. HRF calibration. This calibration can, however, only be done in brain regions that have a tight coupling between these events and evoked neuronal activity. For example, the visual cortex shows a reliable response to visual stimuli. Using fMRI, and the presentation of visual stimuli, the HRF can be deconvolved in the visual cortex and personalized HRF parameters can be derived. With similar methods, the HRF can be estimated for auditory and sensory-motor regions. However, most brain regions do not reliably modulate neuronal activity in response to a stimulus. Consequently, the HRF cannot be calibrated for the majority of the brain.

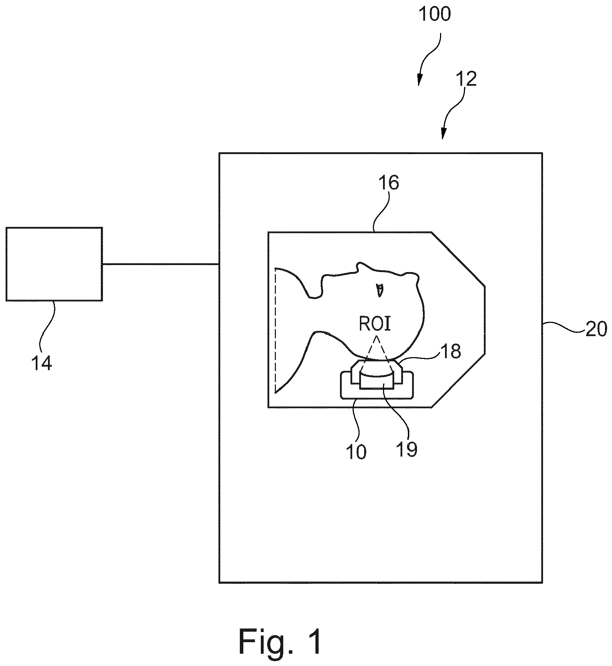

To address at least one of the above-mentioned limitations, Fig. 1 illustrates an example of a system 100 for creating a hemodynamic brain atlas for calibration of brain hemodynamics. The system 100 comprises a non-invasive transcranial neurostimulation device 10, a non-invasive neuromonitoring device 12, and a computing device 14. The target ROI may be e.g. the amygdala, a volume at an atlas coordinate, or a specific part of a brain region (e.g. cortical layer IV of MT in the left hemisphere).

The non-invasive transcranial neurostimulation device 10 is configured to induce neuronal activity to evoke a hemodynamic response in a target region of interest (ROI) of a brain of a human subject.

In the example shown in Fig. 1, the non-invasive transcranial neurostimulation device 10 is a transcranial focused ultrasound (tFUS) device. TFUS is a non-invasive technique to evoke neuronal activity using low intensity ultrasound. TFUS can safely and effectively modulate neuronal activity in animals and humans. Neurostimulation using tFUS requires far less energy as compared to high-focused ultrasound (HIFU), used for ablation. TFUS is not limited to superficial layers of the cortex, but can target deep brain structures as well and can be highly focal. In addition, the effectiveness of tFUS in cortical and deep brain structure is improving with advent of tFUS systems that employ multiple ultrasound transduces, which can focus extra energy in localized deep brain structures.

As shown in Fig. 1, the tFUS device is configured to transcranially deliver pulsed low-intensity FUS to the ROI, whereby similar ranges of frequencies may be employed to modulate human brain activity and to achieve adequate transcranial transmission through the skull. In the example shown in Fig. 1, the tFUS device is positioned inside an MR head coil 16 in an MRI system 20, with the illustration of the sonication path shown as dotted lines. The tFUS may be applied to the ROI through a coupling hydrogel 18, such as polyvinyl alcohol (PVA) hydrogel. The coupling hydrogel 18 may be around the contour of the skin to achieve tight acoustic coupling while maintaining the orientation of the sonication entry as perpendicular as possible to the scalp. In the example of Fig. 1, the tFUS device comprises a single FUS transducer 19 configured to evoke a hemodynamic response in a single target ROI. In other examples (not shown), the tFUS may be configured to evoke a plurality of hemodynamic responses in a plurality of target ROIs. Each hemodynamic response is evoked in a respective target ROI. This may be done by consecutively delivering pulsed low-intensity FUS to multiple ROIs with the single FUS transducer 19 or by simultaneously delivery multiple pulsed low-intensity FUS to multiple ROIs with multiple transducers (not shown). In other words, neurostimulation may target any specified brain region, but also (if desired) to be able to target multiple different brain regions.

Other non-invasive brain stimulation methods (not shown), such as transcranial magnetic stimulation (TMS) and transcranial direct current stimulation (tDCS), may be used instead, or together, with the tFUS to evoke neuronal activity. However, the size of the modulatory area may be large (e.g. on the order of centimetres) while ability to reach specific regions in deep cortical/subcortical areas may be limited compared to tFUS.

The non-invasive neuromonitoring device 12 is configured to monitor the evoked hemodynamic response in the target ROI.

In some examples, functional neuroimaging modalities, such as functional magnetic resonance imaging (fMRI) and positron emission tomography (PET), may be used to characterize the spatial and temporal features of neural responses to FUS-mediated brain stimulation.

In the example shown in Fig. 1, the non-invasive neuromonitoring device 12 comprises an MRI system 20 that provides a high-resolution anatomical MRI that is usable for stereotactic guidance of tFUS. The MRI system 20 may also be used as the non-invasive neuromonitoring device 12. For example, fMRI may be used to measure neuronal activity in response to the stimulus. Functional MRI sequences typically employ the blood oxygen level-dependent (BOLD) contrast to estimate brain activity. Hence, fMRI depends on the response of blood vessels in the brain, and is therefore limited, and temporally filtered, by the hemodynamics of the brain's vasculature.

In some examples (not shown), the non-invasive neuromonitoring device 12 may perform hemodynamic imaging with low intensity focused ultrasound (LIFU) imaging or near-infrared spectroscopy. Both methods could also be combined with MRI for both neuromonitoring and/or stereotactic navigation. In the case of LIFU, the Doppler aspects of the ultrasound imaging may be used to obtain a measure of the (absolute) blood flow in real time. LIFU imaging may also be used to measure the blood flow in real time with high sensitivity, high spatiotemporal resolution (typically 100 $\mu$m and 10 ms) and large field of view (ranging from square cm to tens of square cm). LIFU is sometimes called functional ultrasound imaging. Thereby, LIFU could be used instead, or together, with fMRI measurements, to deconvolve the HRF and improve calibration for novel fMRI or LIFU measurements. Furthermore, using LIFU, it is possible to measure the blood flow in the blood vessel of interest before (Fmin) and at the peak of the tFUS stimulus (Fmax). Whilst LIFU imaging provides absolute values of the flow rate, this could be even improved upon by using anatomical imaging of the size of the blood vessel to calculate the total change in blood volume. For example, in the example of visual stimulus, it can be expected that the size of the blood vessel will change from initial area of blood vessel (Amin) and after 6 seconds reach its largest size (e.g. area of blood vessel Amax). Hence, if a second more dedicated scan (i.e. a higher resolution etc.) is taken after 6 seconds the change in (absolute) blood flow is calculated by

$$V_{max} = V_{min} \times (F_{max} \times A_{\max})/(F_{min} \times A_{min})$$

This is another way to calibrate the BOLD response using LIFU and fMRI measurement. In addition, any change resulting from thermal events (i.e. changes in blood viscosity) caused by the stimulus may also be identified by e.g. using near-infrared spectroscopy.

In some further examples (not shown), the non-invasive neuromonitoring device 12 may measure the brain's electrical currents to estimate the amount of neuronal activity induced by the non-invasive transcranial neurostimulation device 10. Exemplary non-invasive neuromonitoring methods may include, but are not limited to, electroencephalography (EEG), magnetoencephalography (MEG), Optically-Pumped Magnetometers (OPM), and motor-evoked responses. The additional estimates of neuronal activity may be used to infer the expected HRF. These independent estimates of neuronal activity can help further improve the calibration procedure and inform e.g. tFUS parameters, including the intensity, frequency or duration of tFUS.

The computing device 14 is configured to determine a set of parameters representing a hemodynamic response function (HRF) of the evoked hemodynamic response in the target ROI. The computing device 14 is further configured to associate the set of parameters of the HRF with the target ROI to form the hemodynamic brain atlas. The set of parameters representing HRF may include one or more of peak amplitude, trough amplitude, time to peak, time to fall, rise slope, fall slope, FWHM, etc.

When the hemodynamic brain atlas acquired before and after medication, the influence of this medication on several regions or networks may be registered for instance to investigate the effects of medication. This could be used to assess the influence of medication on brain activity, for example psychoactive medicinal drugs. This may enable personal medication to its full potential: each person has different susceptibility for brain medication. Side effects of brain medication may be high and can be predicted at forehand.

An operation example of using a tFUS-MRI system is shown in Figs. 2A-2C. A patient will be placed inside the MRI system 20. First, as shown in Fig. 2A, a high-resolution anatomical MRI is made for stereotactic guidance of tFUS and to register the various measurements. One or more tFUS devices will then be used to evoke a HRF in one or more target ROIs (e.g. the amygdala, a volume at an atlas coordinate, or a specific part of a brain region). During, and immediately following transcranial neurostimulation, the evoked hemodynamic response will be measured using fMRI. This can be compared with baseline measurements without stimulation or sham. The scanner will be optimized to monitor the ROI with a high temporal and spatial resolution in order to sample the shape of the hemodynamic response and filter potential confounders. From each measurement, an HRF can be deconvolved and the function parameters estimated. This procedure may be repeated several times to decrease noise and estimate variance in the HRF. An exemplary HRF deconvolution is shown in Fig. 2B. The HRF deconvolution results in individualized HRF parameters (e.g. peak amplitude, trough amplitude, time to peak, time to fall, rise slope, fall slope, and/or FWHM) for the stimulated brain ROI. Using the information of the anatomical MRI, the HRF parameters will be mapped onto a hemodynamic brain atlas. An exemplary hemodynamic brain atlas is shown in Fig. 2C, which is mapped onto a stereotactic space and stored in a digital file.

In the example of Figs. 2A-2C, there is a single brain region that is determined by the ROI and the spatial resolution of tFUS (and fMRI). The same calibration procedure can be repeated to calibrate the HRF for a series of other brain regions, resulting in a hemodynamic brain atlas.

The regionally calibrated HRFs based on hemodynamic brain atlas may be used during acquisition of novel fMRI measurements. The estimation of new fMRI activity works akin to current statistical parametric mapping studies, but instead of a canonical HRF, the regionally calibrated HRFs are convolved to estimate fMRI activity. Thus, a more accurate estimation of brain activity of the patient is obtained, which supports the formation of a correct conclusion or diagnosis.

Optionally, the target ROI(s) may be selected from a set of calibration brain regions. An HRF in other brain regions of the brain may be derivable from an HRF in the set of calibration brain regions. Although the HRF differs across brain regions, there are regularities (for example between hemispheres). For example, once a sufficient number of patients have been mapped into a hemodynamic brain atlas, these regularities may be exploited, and an optimal set of calibration regions may be defined. For example, following stimulation, the HRF in the entorhinal cortex might on average peak half a second later than the HRF in the hippocampus. Also, in one patient the HRF might have a 20% larger amplitude than another patient. The set of calibration brain regions may take these regularities between brain regions and individuals into account. By calibrating the HRF in a single (or optimal set) of brain regions, the HRF parameters will be inferred for un-calibrated brain region. In this way, the hemodynamic brain atlas will be extended using a transfer function. This means that using a small set of individually calibration regions, for example one in each lobe, the method will infer a whole-brain hemodynamics.

Optionally, the non-invasive neuromonitoring device 12 may be configured to monitor a further evoked hemodynamic response in one or more brain regions that have sufficiently strong excitatory connectivity with the target ROI. The computing device 14 may be configured to determine a set of parameters representing an HRF of the further evoked hemodynamic response in the one or more brain regions, and to associate the set of parameters with the one or more brain regions to form the hemodynamic brain atlas. In other words, the HRF is not only estimated in the stimulated ROI(s), but also in other regions that might show a consistent BOLD response after stimulation. This is because neuronal activity is known to propagate to brain regions with dense excitatory connections. In some instances, neurostimulation might therefore also evoke a neuronal, and ensure BOLD response, in a distal brain region with strong excitatory connectivity. Using the comparison with baseline measurements made without stimulation, the system could calibrate multiple brain regions using neurostimulation of only a single ROI or a small number of ROIs.

Optionally, the non-invasive transcranial neurostimulation device 10 may be configured to evoke a sequence of hemodynamic responses in the target ROI. The non-invasive neuromonitoring device 12 is configured to monitor the sequence of evoked hemodynamic responses in the target ROI. The computing device 14 is configured to perform a comparison among the sequence of evoked hemodynamic responses to correct a potential neurostimulation-induced confound on the HRF. For example, the non-invasive transcranial neurostimulation device may be an MRI. The MRI may use fMRI sequences to better separate the influence of changes in cerebral blood flow and BOLD, such as vascular space occupancy depended fMRI, or a FMR sequence that allows better estimation of the influence of temperature changes and BOLD.

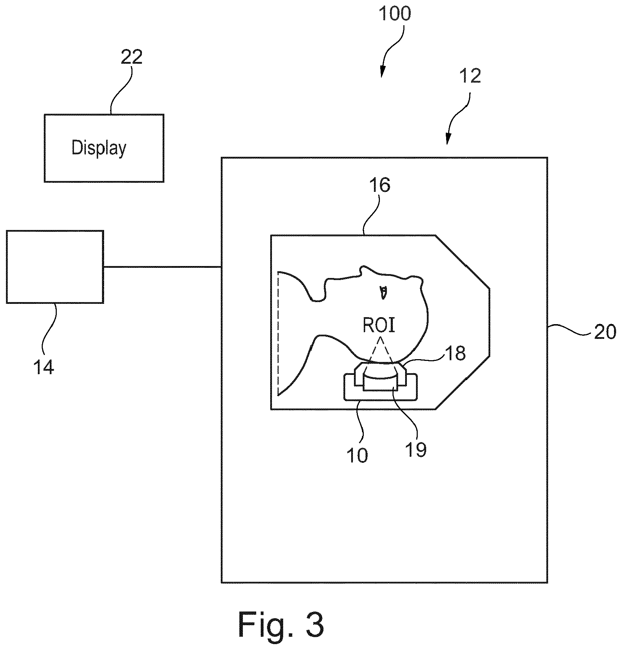

Fig. 3 illustrates a further example of a system 100 for creating a hemodynamic brain atlas for calibration of brain hemodynamics. In this example, the system 100 further comprises a sensory stimulation device 22 configured to apply at least one sensory stimulus over a human subject to induce neuronal activity to evoke a hemodynamic response in the target ROI.

In the example illustrated in Fig. 3, the sensory stimulation device 22 is a visual stimulation device. The visual stimulation device may be a screen, such as a computer monitor or a television, for displaying any combination of still images and video for predetermined durations on predetermined areas of the screen. The visual stimuli may be brought into the scanner using a mirror arrangement to allow the patient to view an (inverted) display in the mirror, by a projection of the visual stimuli onto the bore of the MRI, by providing the patient with MRI compatible head mounted displays etc.

In another example (not shown), the sensory stimulation device 22 may be an auditory stimulation device, such as a speaker connected to an audio source and outputting predetermined sounds at predetermined timings according to the control signal.

In a further example (not shown), the sensory stimulation device 22 may be a tactile stimulation device, such as a vibrating device that is touching (or very close to) the human subject and in response to the control signal, vibrates at predetermined vibration strength and frequency and at predetermined timings.

The computing device 14 may be configured to perform a comparison between the hemodynamic response evoked by the non-invasive transcranial neurostimulation device and the hemodynamic response evoked by the sensory stimulation device to correct a potential neurostimulation-induced confound on the HRF.

In other words, for one single distinct brain region the deconvolution of the neurostimulation-evoked HRF may be compared to a deconvolution of a sensory-evoked HRF. This direct comparison may enable the system to control for neurostimulation-induced confounds on the HRF. Slight tissue heating (within the safety margins) might result in larger HRF response. The calibration procedure includes a comparison of sensory-evoked HRF in the same brain region, thereby creating an additional reference. For example, the visual cortex may be calibrated twice, using visual stimulation via a projector and using tFUS as shown in Fig. 3. The comparison between both types of stimulation will allow for estimation and correction of tFUS induced influence on the HRF.

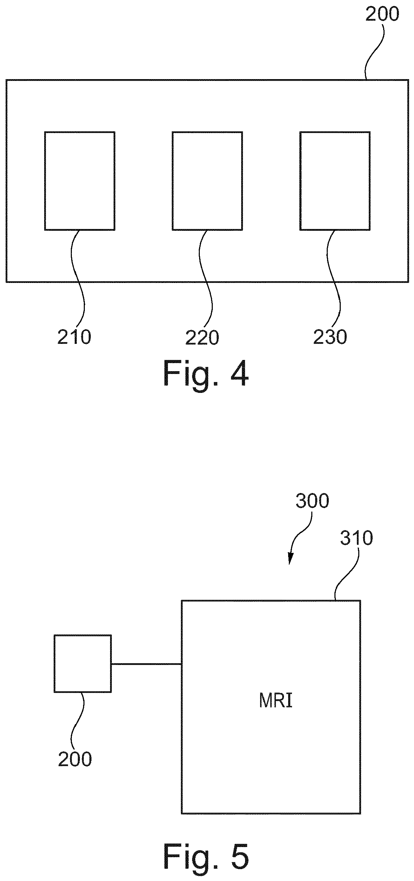

Fig. 4 schematically illustrates a device 200 for calibration of brain hemodynamics. The device 200 comprises an input unit 210, a processing unit 220, and an output unit 230.

The input unit 210 is configured to receive (i) a hemodynamic response in a region of interest (ROI) of a brain of a human subject acquired by a non-invasive neuromonitoring device and (ii) a hemodynamic brain atlas.

In some examples, the hemodynamic response may be obtained using functional neuroimaging modalities, such as fMRI and PET. In some examples, the hemodynamic response may be obtained using hemodynamic imaging with low intensity focused ultrasound imaging or near infrared spectroscopy.

The hemodynamic brain atlas comprises one or more brain regions. Each brain region is associated with a respective hemodynamic response function (HRF) represented by a set of parameters, such as peak amplitude, trough amplitude, time to peak, time to fall, rise slope, fall slope, FWHM, etc. An exemplary hemodynamic brain atlas is illustrated in Fig. 2B.

In some examples, the hemodynamic brain atlas may include parameters based on prior information from measurements made at earlier times for the same patient. For example, it is possible to combine several HRF measurements done on the same brain area of the same person over the course of a number of weeks or years. This may also be considered a diagnostic tool in itself: it might show how the shape and timing of the HRF in a certain brain area changes or deteriorates over time.

In some examples, the hemodynamic brain atlas may include parameters based on prior information from measurements made at earlier times in other patients. Such hemodynamic brain atlas may also be referred to as "external atlas". For example, the hemodynamic brain atlas may be derivable from previous HRF measurements of a group of healthy adults, optionally stratified by age, sex or other factors, previous HRF measurements of a specified patient group, and previous HRF measurements of a single patient for whom may brain regions have been calibrated. This external atlas might also include HRF parameters that have been derived from different individuals for various brain regions. For example, the visual cortex might have been estimated in one patient, but the amygdala in another patient, etc. Before application, the system or an operator, could use various patient characteristics (e.g. age, gender, diagnosis) to select the most appropriate hemodynamic brain atlas.

The input unit 210 is, in an example, implemented as an Ethernet interface, a USB (TM) interface, a wireless interface such as a WiFi (TM) or Bluetooth (TM) or any comparable data transfer interface enabling data transfer between input peripherals and the processing unit 220.

The processing unit 220 is configured to calibrate the acquired hemodynamic response with the hemodynamic response atlas. The calibrated HRF based on hemodynamic brain atlas is used during acquisition of novel fMRI measurements. For example, the estimation of fMRI activity works akin to current statistical parametric mapping studies, but instead of a canonical HRF, the regionally calibrated HRFs are convolved to estimate fMRI activity. Thus, a more accurate estimation of brain activity of the patient is obtained, which supports the formation of a correct conclusion or diagnosis.

In some examples, the target ROI(s) may be different from the one or more brain regions of the hemodynamic brain atlas. In other words, the calibrated HRF in the hemodynamic brain atlas may be applied to other brain regions than those that showed a response during calibration. For example, a hemodynamic brain atlas with a set of calibrated ROIs from the left hemisphere may be used to estimate brain activity in the contra-lateral hemisphere. During novel fMRI acquisition, a larger set of regions may be used that include the brain regions in the contra-lateral hemisphere, regardless of whether these additional regions showed an evoked BOLD response following stimulation of the ROIs.

The processing unit 220 may refer to, be part of, or include an Application Specific Integrated Circuit (ASIC), an electronic circuit, a processor (shared, dedicated, or group) and/or memory (shared, dedicated, or group) that execute one or more software or firmware programs, a combinational logical circuit, and/or other suitable components that provide the described functionality. Furthermore, such processing unit 14 may be connected to volatile or non-volatile storage, display interfaces, communication interfaces and the like as known to a person skilled in the art.

The output unit 230 is configured to output the calibrated hemodynamic response.

Fig. 5 schematically shows an example of a neuromonitoring system 300. The neuromonitoring system 300 comprises a non-invasive neuromonitoring device 310 and a device 200 as described with respect to Fig. 4.

The non-invasive neuromonitoring device 310 is configured for acquiring a hemodynamic response in a region of interest (ROI) of a brain of a human subject.

In the example of Fig. 5, the non-invasive neuromonitoring device 310 is an MRI device. In other examples (not shown), the non-invasive neuromonitoring device 310 may perform functional neuroimaging with PET.

The device 200 is configured for calibrating the acquired hemodynamic response, e.g. by convolving regionally calibrated HRFs in the hemodynamic brain atlas to estimate fMRI activity.

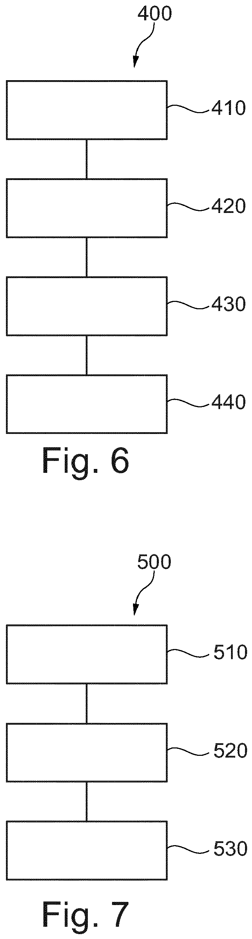

Fig. 6 illustrates a flowchart of a method 400 for creating a hemodynamic brain atlas for calibration of brain hemodynamics.

In step 410, a non-invasive transcranial neurostimulation device induces neuronal activity to evoke a hemodynamic response in a target region of interest (ROI) of a brain of a human subject.

The non-invasive transcranial neurostimulation device may include one or more of tFUS, tDCS/tACS, and TMS.

The target ROI may be anatomically, functionally or clinically defined. The ROI can be anatomically defined as the motor cortex, visual cortex, amygdala, a volume at an atlas coordinate, or a specific part of a brain region in the cortex (e.g. cortical layer IV of MT in the left hemisphere), sub-cortical region (e.g. substantia nigra, thalamus, hippocampus), or a region in the cerebellum. The ROI can be functionally defined using and localizer task in the fMRI (motor, language, etc.), using functional connectivity, a functional atlas or using a PET tracer. The ROI can be clinically, for example as the foci of epileptic seizures or a stroke.

If desired, the non-invasive transcranial neurostimulation device may target multiple different brain regions consecutively with a single transducer or at the same time by multiple transducers.

In step 420, a non-invasive neuromonitoring device monitors the evoked hemodynamic response in the target ROI.

The non-invasive neuromonitoring device may include one or more of fMRI, PET, Functional Ultrasound, LIFU, NIRS, EEG, MEG, and OPM.

In step 430, a computing device determines a set of parameters representing an HRF of the evoked hemodynamic response in the target ROI. The set of parameters may include one or more of peak amplitude, trough amplitude, time to peak, time to fall, rise slope, fall slope, FWHM, etc.

In step 440, the computing device associates the set of parameters of the HRF with the target ROI to form the hemodynamic brain atlas. The hemodynamic brain atlas thus comprises multiple regionally calibrated HRFs.

Fig. 7 shows a flowchart of a method 500 for calibration of brain hemodynamics.

In step 510, an input unit receives a hemodynamic response in a region of interest (ROI) of a brain of a human subject acquired by a non-invasive neuromonitoring device and (ii) a hemodynamic brain atlas. The hemodynamic brain atlas comprises one or more brain regions, each brain region being associated with a respective hemodynamic response function (HRF) represented by a set of parameters.

In some examples, the hemodynamic brain atlas may include parameters based on prior information from measurements made at earlier times for the same patient.

In some examples, the hemodynamic brain atlas may include parameters based on prior information from measurements in other patients, which is also referred to as external atlas. This external hemodynamic brain atlas could consist of an average from a group of healthy adults, from a specified patient group, or from a single patient for whom many brain regions have been calibrated.

In step 520, a processing unit calibrates the acquired hemodynamic response with the hemodynamic brain atlas.

In step 530, an output unit outputs the calibrated hemodynamic response.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

**Claims**

1. A system (100) for creating a hemodynamic brain atlas for calibration of brain hemodynamics, comprising:

     - a non-invasive transcranial neurostimulation device (10) configured to induce neuronal activity to evoke a hemodynamic response in a target region of interest, ROI, of a brain of a human subject;
     - a non-invasive neuromonitoring device (12) configured to monitor the evoked hemodynamic response in the target ROI; and
     - a computing device (14) configured to determine a set of parameters representing a hemodynamic response function, HRF, of the evoked hemodynamic response in the target ROI, and to associate the set of parameters of the HRF with the target ROI to form the hemodynamic brain atlas.

2. System according to claim 1,
   wherein the non-invasive transcranial neurostimulation device is configured to evoke a plurality of hemodynamic responses in a plurality of target ROIs, each hemodynamic response being evoked in a respective target ROI; and wherein the computing device is configured to determine, for each evoked hemodynamic response, a respective set of parameters representing the corresponding HRF and to associate each set of parameters with the corresponding target ROI to form the hemodynamic brain atlas.

3. System according to claim 1 or 2,
   wherein the non-invasive neuromonitoring device is configured to monitor a further evoked hemodynamic response in one or more brain regions that have sufficiently strong excitatory connectivity with the target ROI; and wherein the computing device is configured to determine a set of parameters representing an HRF of the further evoked hemodynamic response in the one or more brain regions, and to associate the set of parameters with the one or more brain regions to form the hemodynamic brain atlas.

4. System according to any one of the preceding claims, further comprising:

     - a sensory stimulation device (22) configured to apply at least one sensory stimulus over a human subject to induce neuronal activity to evoke a hemodynamic response in the target ROI,
     wherein the computing device is configured to perform a comparison between the hemodynamic response evoked by the non-invasive transcranial neurostimulation device and the hemodynamic response evoked by the sensory stimulation device to correct a potential neurostimulation-induced confound on the HRF.

5. System according to any one of claims 1 to 3,
   wherein the non-invasive transcranial neurostimulation device is configured to evoke a sequence of hemodynamic responses in the target ROI;
   wherein the non-invasive neuromonitoring device is configured to monitor the sequence of evoked hemodynamic responses in the target ROI; and
   wherein the computing device is configured to perform a comparison among the sequence of evoked hemodynamic responses to correct a potential neurostimulation-induced confound on the HRF.

6. System according to any one of the preceding claims,
   wherein the target ROI is selected from a set of calibration brain regions; and wherein an HRF in other brain regions of the brain is derivable from an HRF in the set of calibration brain regions.

7. System according to any one of the preceding claims,
   wherein the non-invasive transcranial neurostimulation device comprises one or more of:

     - transcranial functional ultrasound stimulation, tFUS;
     - transcranial electrical stimulation, tDCS/tACS; and
     - transcranial magnetic stimulation, TMS.

8. System according to any one of the preceding claims,
   wherein the non-invasive neuromonitoring device comprises one or more of:

     - a device for neuromonitoring of brain hemodynamics; and
     - a device for measuring electrical signals produced by neurons to measure brain activity.

9. A device (200) for calibration of brain hemodynamics, comprising:

- an input unit (210) configured to receive (i) a hemodynamic response in a region of interest, ROI, of a brain of a human subject acquired by a non-invasive neuromonitoring device and (ii) a hemodynamic brain atlas, wherein the hemodynamic brain atlas comprises one or more brain regions, each brain region being associated with a respective hemodynamic response function, HRF, represented by a set of parameters;
- a processing unit (220) configured to calibrate the acquired hemodynamic response with the hemodynamic brain atlas; and
- an output unit (230) configured to output the calibrated hemodynamic response.

10. Device according to claim 9, wherein the hemodynamic brain atlas is derivable from one or more of:

- previous HRF measurements of the human subject;
- previous HRF measurements of a group of healthy adults;
- previous HRF measurements of a specified patient group; and
- previous HRF measurements of a single patient for whom brain regions may have been calibrated.

11. Device according to claim 9 or 10, wherein the ROI is different from the one or more brain regions of the hemodynamic brain atlas.

12. A neuromonitoring system (300), comprising:

- a non-invasive neuromonitoring device (310) for acquiring a hemodynamic response in a region of interest, ROI, of a brain of a human subject; and
- a device according to any one of claims 9 to 11 for calibrating the acquired hemodynamic response.

13. A method (400) for creating a hemodynamic brain atlas for calibration of brain hemodynamics, comprising:

- inducing (410), by a non-invasive transcranial neurostimulation device, neuronal activity to evoke a hemodynamic response in a target region of interest, ROI, of a brain of a human subject;
- monitoring (420), by a non-invasive neuromonitoring device, the evoked hemodynamic response in the target ROI;
- determining (430), by a computing device, a set of parameters representing a hemodynamic response function, HRF, of the evoked hemodynamic response in the target ROI; and
- associating (440), by the computing device, the set of parameters of the HRF with the target ROI to form the hemodynamic brain atlas.

14. A computer-implemented method (500) for calibration of brain hemodynamics, comprising:

- receiving (510), by an input unit, (i) a hemodynamic response in a region of interest, ROI, of a brain of a human subject acquired by a non-invasive neuromonitoring device and (ii) a hemodynamic brain atlas, wherein the hemodynamic brain atlas comprises one or more brain regions, each brain region being associated with a respective hemodynamic response function, HRF, represented by a set of parameters;
- calibrating (520), by a processing unit, the acquired hemodynamic response with the hemodynamic brain atlas; and
- outputting (530), by an output unit, the calibrated hemodynamic response.

15. A computer program element for controlling a system according to any one of claims 1 to 8 or a device according to any one of claims 9 to 11, which when being executed by a processor is configured to carry out the method according to claim 13 or 14.

Fig. 1

Hemodynamic brain atlas

| Region | Parameter 1 | Parameter 2 |
|--------|-------------|-------------|
| Amydala | X[t] = 7.21 | Y... |
| Thalamus | X[t] = 5.42 | ... |
| ... | ... | ... |

Fig. 2C

HRF deconvolution

Fig. 2B

tFUS-MRI

ROI

Fig. 2A

Fig. 3

Fig. 4

Fig. 5

Fig. 6

Fig. 7

Europäisches
Patentamt

European
Patent Office

Office européen
des brevets

# EUROPEAN SEARCH REPORT

Application Number

EP 20 21 4003

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/340260 A1 (CHOWDHURY SHUBHAJIT ROY [IN] ET AL) 30 November 2017 (2017-11-30) * paragraphs [0029] - [0053]; figures 2,6-7 * ----- | 1-15 | INV. A61B5/00 A61N1/04 A61N1/36 A61N7/00 |
| X | US 2014/058247 A1 (KAWASHIMA YASUHIRO [JP] ET AL) 27 February 2014 (2014-02-27) * paragraphs [0097] - [0104]; claim 1; figure 3 * ----- | 1,9,13, 14 | A61N5/00 A61N2/00 A61B5/055 A61B5/242 A61B5/377 |
| X | SUH M ET AL: "Blood volume and hemoglobin oxygenation response following electrical stimulation of human cortex", NEUROIMAGE, ELSEVIER, AMSTERDAM, NL, vol. 31, no. 1, 15 May 2006 (2006-05-15), pages 66-75, XP024906484, ISSN: 1053-8119, DOI: 10.1016/J.NEUROIMAGE.2005.11.030 [retrieved on 2006-05-15] * abstract * ----- | 1,9,13, 14 | A61B6/00 A61B8/06 G16H30/40 |
| A | US 2009/156955 A1 (JUNG EDWARD K Y [US] ET AL) 18 June 2009 (2009-06-18) * paragraph [0034] * ----- | 1-15 | |

TECHNICAL FIELDS SEARCHED (IPC)

A61B
A61N
G16H

The present search report has been drawn up for all claims

| Place of search | Date of completion of the search | Examiner |
|---|---|---|
| Munich | 27 May 2021 | Gentil, Cédric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 21 4003

This annex lists the patent family members relating to the patent documents cited in the above-mentioned European search report.
The members are as contained in the European Patent Office EDP file on
The European Patent Office is in no way liable for these particulars which are merely given for the purpose of information.

27-05-2021

| Patent document cited in search report | | | Publication date | Patent family member(s) | | | Publication date |
|---|---|---|---|---|---|---|---|
| US 2017340260 | A1 | | 30-11-2017 | AU | 2016206634 | A1 | 10-08-2017 |
| | | | | BR | 112017015175 | A2 | 23-01-2018 |
| | | | | CA | 2973657 | A1 | 21-07-2016 |
| | | | | CN | 107405480 | A | 28-11-2017 |
| | | | | EP | 3244964 | A1 | 22-11-2017 |
| | | | | JP | 2018508321 | A | 29-03-2018 |
| | | | | KR | 20170128232 | A | 22-11-2017 |
| | | | | US | 2017340260 | A1 | 30-11-2017 |
| | | | | WO | 2016115392 | A1 | 21-07-2016 |
| US 2014058247 | A1 | | 27-02-2014 | JP | 2014039640 | A | 06-03-2014 |
| | | | | US | 2014058247 | A1 | 27-02-2014 |
| US 2009156955 | A1 | | 18-06-2009 | NONE | | | |

EPO FORM P0459

For more details about this annex : see Official Journal of the European Patent Office, No. 12/82